(19) Europäisches Patentamt
European Patent Office
Office européen des brevets

(11) Publication number: 0 268 267 A2

(12) **EUROPEAN PATENT APPLICATION**

(21) Application number: 87116968.6

(22) Date of filing: 17.11.87

(51) Int. Cl.[4]: C07C 129/12 , A61K 31/155

(30) Priority: 17.11.86 JP 271748/86

(43) Date of publication of application: 25.05.88 Bulletin 88/21

(84) Designated Contracting States: AT BE CH DE ES FR GB GR IT LI LU NL SE

(71) Applicant: NIPPON HYPOX LABORATORIES INCORPORATED
1759, Matsugaya Hachioji-shi
Tokyo 192-03(JP)

Applicant: Satoh, Toshio
57-3, Nagao Joroku-cho
Tokushima-shi Tokushima-ken(JP)

(72) Inventor: Satoh, Toshio
57-3, Nagao Jorokucho
Tokushima-City,Tokushima-Pref.(JP)

(74) Representative: Hansen, Bernd, Dr.rer.nat. et al
Hoffmann, Eitle & Partner Patentanwälte
Arabellastrasse 4
D-8000 München 81(DE)

(54) **Guanidinomethylbenzoic acid derivatives, compositions containing the same, processes for preparing the same, and the use of the same in the manufacture of medicaments of therapeutic value.**

(57) A guanidinomethylbenzoic acid derivative or a salt thereof of the invention having excellent antiulcer activity and a low toxicity is provided. Pharmaceutical compositions containing such compounds are also provided.

The invention also includes processes for their preparation and the use of such compounds for the manufacture of medicaments of therapeutic value.

EP 0 268 267 A2

# GUANIDINOMETHYLBENZOIC ACID DERIVATIVES, COMPOSITIONS CONTAINING THE SAME, PROCESSES FOR PREPARING THE SAME, AND THE USE OF THE SAME IN THE MANUFACTURE OF MEDICAMENTS OF THERAPEUTIC VALUE

## BACKGROUND OF THE INVENTION

### Field of the Invention

The present invention relates to guanidinomethylbenzoic acid derivatives, and also to pharmaceutical compositions comprising said derivatives as an effective ingredient.

### Prior Art

A number of compounds have heretofore been proposed for use in the treatment and prevention of gastroenteric ulcers. For example, N-(phenyl)-trans-4-guanidinomethylcyclohexanecarboxamide hydrochloride represented by the following formula is disclosed in Japanese Laid-Open (Kokai) No. 57-197256 as a compound having an antiulcer activity.

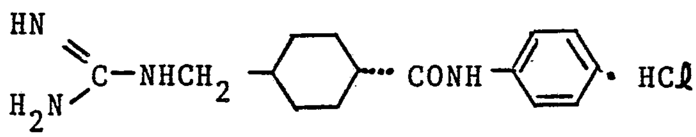

This compound, however, is not satisfactory in antiulcer activity and has a relatively high toxicity.

Hence, an object of the present invention is to provide novel compounds having an excellent antiulcer activity and a low toxicity.

Another object of the present invention is to provide antiulcer agents comprising said compounds having said properties as an effective ingredient.

Other objects of the present invention will become apparent from the following description.

## SUMMARY OF THE INVENTION

In order to achieve the above objects, the present inventors synthesized various compounds and examined their antiulcer activities. As a result, it was found that as is clear from the experimental results given later, guanidinomethylbenzoic acid derivatives represented by the following general formula (I) as well as their salts exhibit an excellent antiulcer activity and a very low toxicity, both of which characteristics can not be predicted from a known antiulcer agent, namely, N-(phenyl)-trans-4-guanidinomethylcyclohexanecarboximide hydrochloride.

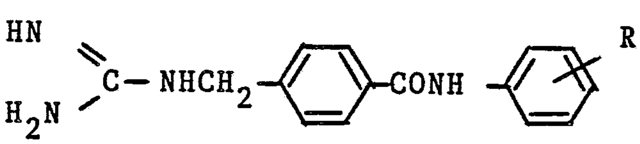

(I)

(wherein R is a member selected from the group consisting of a halogen atom, a hydroxyl group, a lower alkyl group, a lower alkoxy group and a lower alkyl-substituted or unsubstituted carboxyl group)

Accordingly, the present invention relates to guanidinomethylbenzoic acid derivatives of the general formula (I) and their salts, as well as to pharmaceutical compositions comprising at least one of said derivatives and salts as an effective ingredient. Such compositions are effective agents against ulcers.

DETAILED DESCRIPTION OF THE INVENTION

The compounds of the present invention include the compounds represented by the general formula (I) and organic or inorganic salts thereof. As such salts, there can be mentioned, for example, salts of an inorganic acid (e.g. hydrochloric acid, hydrobromic acid, sulfuric acid, nitric acid, phosphoric acid) or of an organic acid (e.g. acetic acid, propionic acid, citric acid, lactic acid, tartaric acid, p-toluenesulfonic acid). Particularly preferred among these salts are pharmaceutically acceptable salts.

In the present specification, the term "lower" refers to that a group or compound to which said term is attached has carbon atoms of 5 or less, preferably 3 or less. Accordingly, "a lower alkyl group" in the general formula (I) refers to a straight chain or branched chain alkyl group having 5 or less carbon atoms; and as such a lower alkyl group, there can be mentioned, for example, a methyl group, an ethyl group, an n-or iso-propyl group and an n-, iso or t-butyl group, among which the methyl group being preferred.

"A lower alkoxy group" in the general formula (I) refers to an alkoxy group having a straight chain or branched chain alkyl group having 5 or less carbon atoms; and as such a lower alkoxy group, there can be mentioned, for example, a methoxy group, an ethoxy group and an n-or isopropoxy group. "A lower alkyl-substituted or unsubstituted carboxyl group" includes a methoxycarbonyl group, an ethoxycarbonyl group, an unsubstituted carboxyl group, etc. "A halogen atom" refers to a fluorine atom, a chlorine atom, a bromine atom or an iodine atom.

Typical examples of the guanidinomethylbenzoic acid derivatives of the general formula (I) provided by the present invention include the following compounds, in addition to the compounds mentioned in Examples which appear later.

N-(2′-chlorophenyl)-4-guanidinomethylbenzamide
N-(2′-fluorophenyl)-4-guanidinomethylbenzamide
N-(2′-methylphenyl)-4-guanidinomethylbenzamide
N-(3′-ethylphenyl)-4-guanidinomethylbenzamide
N-(3′-butylphenyl)-4-guanidinomethylbenzamide
N-(4′-ethoxyphenyl)-4-guanidinomethylbenzamide
N-(2′-ethoxyphenyl)-4-guanidinomethylbenzamide
N-(4′-bromophenyl)-4-guanidinomethylbenzamide
N-(4′-methoxycarbonylphenyl)-4-guanidinomethylbenzamide
N-(4′-propylphenyl)-4-guanidinomethylbenzamide
N-(4′-pentylphenyl)-4-guanidinomethylbenzamide, etc.

According to the present invention, the guanidinomethylbenzoic acid derivative represented by the general formula (I) or the salt thereof can be produced, for example, by reacting 4-guanidinomethylbenzoic acid (II) with an aniline derivative (III) in the presence of dicyclohexylcarbodiimide (hereinafter referred to as DCC) or phosphorus trichloride ($PCl_3$) and, as necessary, converting the resulting free product to a salt thereof.

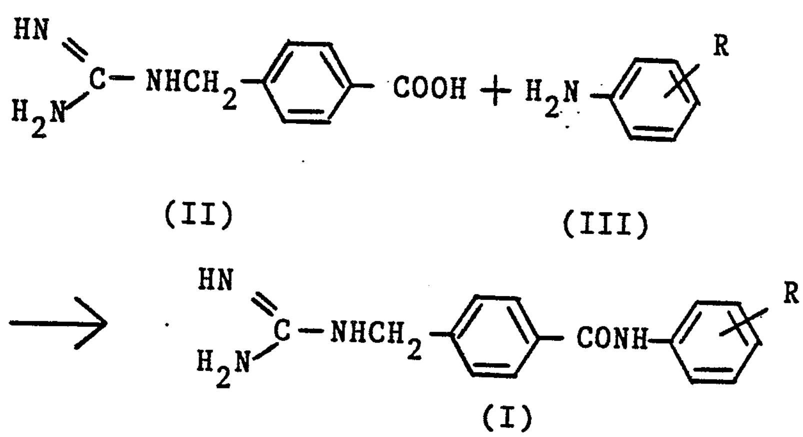

(wherein R has the same definition as given previously).

That is, a desired compound (I) can be obtained by a compound (II) and a compound (III) being subjected to dehydrating condensation in the presence of DCC or $PCl_3$. The amount of the compound (III) used is 1.0 to 1.5 moles per 1.0 mole of the compound (II). The amount of DCC or $PCl_3$ used is 1.0 to 1.5 moles per 1.0 mole of the compound (II). As preferable solvent, there can be mentioned, for example, acetone, tetrahydrofuran, dioxane and pyridine, all of which are anhydrous. The reaction completes by

stirring for 10 to 80 hours at room temperature or by refluxing for 1 to 5 hours. After completion of the reaction, insoluble materials are removed from the reaction mixture by filtration; the filtrate is concentrated to dryness; and the residue is purified to obtain a desired compound (I).

The obtained guanidinomethylbenzoic acid derivative of the general formula (I) can as necessary be converted to a corresponding salt, by treating said derivative with, for example, an inorganic acid (e.g. hydrochloric acid, hydrobromic acid, sulfuric acid, nitric acid, phosphoric acid) or an organic acid (e.g. acetic acid, propionic acid, citric acid, lactic acid, tartaric acid) according to a method known per se.

In the above reaction, the compound (II) or (III) in the form of salt can be used under the nonbasic condition to directly obtain the compound (I) in the form of salt.

The desired compound (I) in the form of a salt may as necessary be converted to another salt according to a conventional salt exchanging method. The compounds (I) or their salts are applicable as an antiulcer agent to the treatment of human beings and warm-blooded animals by oral or parenteral administration (e..g. intramuscular injection, subcutaneous injection, local administration, etc.).

The compounds of the present invention, when used as a drug, can be prepared into various forms suitable for oral or parenteral administration. For example, they can be prepared into drugs in combination with pharmaceutically acceptable, nontoxic carriers for common use. These drugs can be prepared in any of solid forms (e.g. tablets, capsules, granules, powders, fine powders, sugar coated pills and troches, etc.), semi-solid forms (e.g. ointments, creams, suppositories, etc.) and liquid forms (e.g. injections, emulsions, suspensions, lotions, tinctures, sprays and syrups, etc.), depending upon the route by which the drugs are used. Suitable pharmaceutically acceptable, nontoxic carriers include, for example, starch, gelatin, grape sugar, lactose, fruit sugar, maltose, magnesium carbonate, talc, magnesium stearate, methyl cellulose, carboxymethyl cellulose (CMC) and its salts, gum arabic, polyalkylene glycols, distilled water for injection use, alkyl p-hydroxybenzoate, syrups, ethanol, propylene glycol, glycerin, petrolatum, carbowax and the like.

The aforesaid drugs may contain other therapeutically useful agents, dispersants, antioxidants, preservatives, stabilizers, perfumes, binders, lubricants, osmotic pressure regulating salts, buffers and the like.

The amount of the compound of the present invention in a drug varies with the form of the drug but is preferably in the range of 5 to 100% by weight in the case of solid and semi-solid drugs and in the range of 0.1 to 10% by weight in the case of liquid drugs.

The dose of the compound of the present invention varies widely depending upon, for example, individual patients, the kinds of warm-blooded animals to be treated, disease conditions and diagnoses made by doctors. The compound can be applied usually in a daily dose of 0.01 to 30 mg/kg, preferably 0.1 to 20 mg/kg. Even doses out of the range are applicable according to the disease conditions or the doctors' diagnoses. The above mentioned daily dose can be administered at one time or plural times in a day.

The guanidinomethylbenzoic acid derivatives of the general formula (I) or their salts according to the present invention are capable of exhibiting excellent antiulcer activities as evidenced by the following animal tests.

The animal tests were conducted using the following compounds each given a symbol.

## Test Compounds

A: N-(4'-methylphenyl)-4-guanidinomethylbenzamide hydrochloride
B: N-(4'-chlorophenyl)-4-guanidinomethylbenzamide hydrochloride
C: N-(2'-ethoxycarbonylphenyl)-4-guanidinomethylbenzamide hydrochloride
D: N-(2'-methoxyphenyl)-4-guanidinomethylbenzamide hydrochloride
E: N-(phenyl)-trans-4-guanidinomethyl-cyclohexanecarboximide hydrochloride (comparative compound)

## Test Method 1 (Effect on Stress Induced Ulcer)

1. Test Procedure

The method of Takagi et al. (Jap. J. Pharmacol., 18, 9 (1986)) was followed. Male rats of Sprague Dawley (SD) strain each weighing from 180 to 200 g (8 weeks old and 6 to 8 rats in one group) were fasted for 24 hours, followed by oral administration of the above test compounds which had been suspended in 1% CMC aqueous solution. Fifteen minutes later, the animals were placed in a stress cage which was then

immersed in a water bath at 24°C at the depth of the xiphisterna of the animals. After being immersed for 18 hours, the animals were sacrificed under etherization to remove the stomachs. Each of the stomachs was injected with 12 ml of 1% formaldehyde solution and then immersed in a 1% formaldehyde solution for 15 minutes. The stomach was incised along its greater curvature and examined for the gastric lesions under a stereomicroscope for the major axis (mm) of each ulcer that had developed on the gastric mucous membrane. The total major axis (mm) per animal was taken as the ulcer index of the animal. The ulcer inhibition rate was calculated from the following equation. The $ED_{50}$ value was obtained from the dose-ulcer inhibition rate curve.

Ulcer inhibition rate (%) = $(1 - \frac{m}{\ell}) \times 100$

(wherein $\ell$ is the ulcer index of a group without the test compounds administered, and m is the ulcer index of a group having such compounds administered).

2. Test results

The results are shown in Table 1.

Table 1

| Compound | Dose (mg/kg) | Ulcer index (mm) | Ulcer inhibition rate (%) |
|---|---|---|---|
| A | 100 | 9.2 ± 3.4 | 90.3 |
| B | 100 | 18.4 ± 8.8 | 80.5 |
| C | 100 | 15.0 ± 14.7 | 84.1 |
| D | 100 | 23.8 ± 14.9 | 74.8 |
| E (comparative) | 100 | 61.4 ± 14.0 | 35.0 |
| Control | 0 | 94.4 ± 25.1 | 0 |

As is clearly evident from the results given in Table 1, the compounds A, B, C and D which are the typical examples of the claimed compound, show a remarkable antiulcer effect, which is superior to that of the compound E , known anti-ulcer agent mentioned in the Prior Art section.

Test Method 2 (Acute Toxicity - Minimum Lethal Dose (MLD))

1. Test Procedure

Male mice of ddY strain each weighing from 20 to 22 g (4 weeks old and 5 mice in one group) were fasted overnight, followed by oral administration of the test compounds which had been suspended in 1% CMC aqueous solution. Observation was made over 7 days to determine the minimum lethal dose (MLD).

2. Test Results

All of the test compounds A to D showed a MLD of 3,000 mg/kg or more.

In contrast, the comparative compound E showed a MLD of 1,000 mg/kg or below.

From these results, it is evident that the claimed compound has a lower toxicity than the comparative compound.

The invention will now be further described by way of the following examples.

Example 1 Production of N-(4'-methylphenyl)-4-guanidinomethylbenzamide hydrochloride (compound A previously shown)

p-Toluidine (5.0 g, 46.7 mmol) dissolved in pyridine (50 ml) was cooled to below 0°C. Thereto was dropwise added $PCl_3$ (3.2 g, 23.4 mmol) dissolved in pyridine (20 ml).

Fifteen minutes later, the mixture was returned to room temperature and 4-guanidinomethylbenzoic acid hydrochloride (10.8 g, 46.7 mmol) was added. The resulting mixture was reacted for 3 hours, and concentrated. The residue was poured into ice water to precipitate crude crystals.

The crude crystals were recrystallized from $H_2O$ to obtain 7.9 g of the captioned compound as white crystals.

mp: 124-125°C

IR (KBr) $\nu$max ($cm^{-1}$): 3400-3000, 1665, 1600

MS (m/e): 282 ($M^+$ -HCl)

Example 2 Production of N-(4'-chlorophenyl)-4-guanidinomethylbenzamide hydrochloride (compound B previously shown)

4-guanidinomethylbenzoic acid hydrochloride (mp: above 270°C) (1.0 g, 4.3 mmol) and p-chloroaniline (0.62 g, 4.8 mmol) were dissolved in pyridine (50 ml) and DMF (20 ml). DCC (1.0 g, 4.9 mmol) was added thereto and the mixture was reacted for 100 hours at room temperature. After completion of the reaction, the reaction mixture was filtered. The filtrate was concentrated and the residue was added to cold water (50 ml). The resulting crystal was removed by filtration, and the filtrate was concentrated to dryness. The residue was washed with benzene (50 ml) and ethyl acetate (50 ml) in this order and then recrystallized from water-methanol (1:1) to obtain 0.25 g of the captioned compound as white crystals.

mp: 199-202°C

IR (KBr) $\nu$max ($cm^{-1}$): 3400-3000, 1680, 1600

MS (m/e): 302 ($M^+$ -HCl)

Example 3 Production of N-(2'-ethoxycarboxylphenyl)-4-guanidinomethylbenzamide hydrochloride (compound C previously shown)

4-guanidinomethylbenzoic acid hydrochloride (mp: above 270°C) (1.0 g, 4.3 mmol) and ethyl anthranilate (0.83 g, 5.0 mmol) were dissolved in pyridine (50 ml) and DMF (20 ml). DCC (1.0 g, 4.9 mmol) was added thereto and the mixture was reacted for 100 hours at room temperature. After completion of the reaction, the reaction mixture was filtered and the filtrate was concentrated. The residue was dissolved in hot water and the solution was extracted with ethyl acetate. After the ethyl acetate layer was concentrated to dryness, the residue was recrystallized from water to obtain 0.21 g of the captioned compound as white crystals.

mp: 211-213°C

IR (KBr) $\nu$max ($cm^{-1}$): 3400-3000, 1670, 1600

MS (m/e): 340 ($M^+$ -HCl)

The following compounds were produced in the same manner as in Example 1.

Example 4 N-(2'-methoxycarbonylphenyl)-4-guanidinomethylbenzamide hydrochloride mp: 120-122°C
IR (KBr) νmax ($cm^{-1}$): 3400-3100, 1675, 1600
MS (m/e): 326 ($M^+$ -HCl)

Example 5 N-(4'-hydroxyphenyl)-4-guanidinomethylbenzamide hydrochloride mp: above 300°C
IR (KBr) νmax ($cm^{-1}$): 3400-3100, 1690, 1600
MS (m/e): 284 ($M^+$ -HCl)

Example 6 N-(4'-methoxyphenyl)-4-guanidinomethylbenzamide hydrochloride (compound D previously shown) mp: 260-263°C
IR (KBr) νmax ($cm^{-1}$): 3400-3000, 1650, 1600
MS (m/e): 298 ($M^+$ -HCl)

Example 7 N-(4'-t-butylphenyl)-4-guanidinomethylbenzamide hydrochloride mp: 254-256°C
IR (KBr) νmax ($cm^{-1}$): 3400-3000, 1650, 1600
MS (m/e): 324 ($M^+$ -HCl)

## Claims

1. A guanidinomethylbenzoic acid derivative represented by the following general formula:

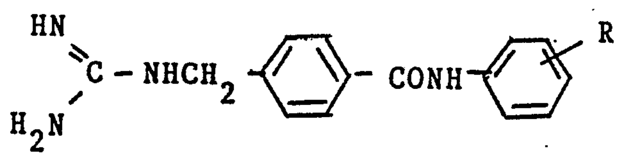

(I)

wherein R is a member selected from the group consisting of a halogen atom, a hydroxyl group, a lower alkyl group, a lower alkoxy group and a lower alkyl-substituted or unsubstituted carboxyl group, or a salt thereof.

2. A guanidinomethylbenzoic acid derivative or a salt thereof according to Claim 1, wherein R represents a chlorine atom.

3. A guanidinomethylbenzoic acid derivative or a salt thereof according to Claim 1, wherein R represents a methyl group.

4. A guanidinomethylbenzoic acid derivative or a salt thereof according to Claim 1, wherein R represents a methoxy group.

5. A guanidinomethylbenzoic acid derivative or a salt thereof according to Claim 1, wherein R represents an ethoxycarbonyl group.

6. A pharmaceutical composition comprising a guanidinomethylbenzoic acid derivative according to any preceding claim and a pharmaceutically acceptable carrier.

7. A composition according to Claim 6 for use as an anti-ulcer agent.

8. A process for preparing a guanidinomethylbenzoic acid derivative as claimed in any of Claims 1-5 comprising the step of condensing together the compound of formula II, or optionally a salt thereof, and a compound of formula III, or optionally, a salt thereof, in the presence of a dehydrating agent, and optionally converting the product into a salt.

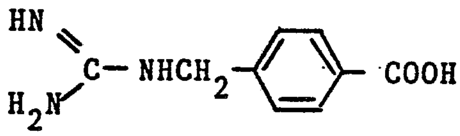

(II)

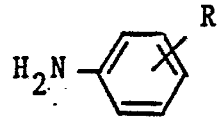

(III)

wherein R is as defined in claim 1.

9. A process according to Claim 8 wherein the molar ratio of the compound of formula II to the compound of formula III is 1.0:1.0-1.5.

10. A process according to Claim 8 or Claim 9 wherein the dehydrating agent is dicyclohexylcarboiimide or phosphorus trichloride.

11. A process according to Claim 10 wherein the molar ratio of the compound of formula II to the dehydrating agent is 1.0:1.0-1.5.

12. A process according to any of Claims 8-11 wherein the condensation reaction is carried out for a period of from 10 to 80 hours at room temperature.

13. A process according to any of Claims 8-11 wherein the condensation reaction is carried out for a period of from 1 to 5 hours under reflux.

14. The use of a guanidinomethylbenzoic acid derivative of general formula:

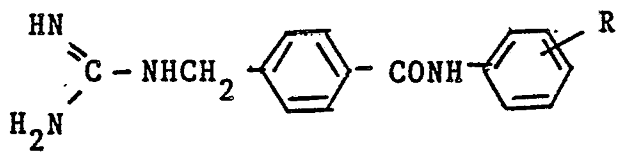

(I)

wherein R is a member selected from the group consisting of a halogen atom, a hydroxyl group, a lower alkyl group, a lower alkoxy group and a lower alkyl-substituted or unsubstituted carboxyl group,
or a salt thereof,
for the manufacture of a medicament for the treatment of ulcers.

Claims for the following contracting States: ES, GR

1. A process for preparing a guanidinomethylbenzoic acid derivative represented by the following general formula:

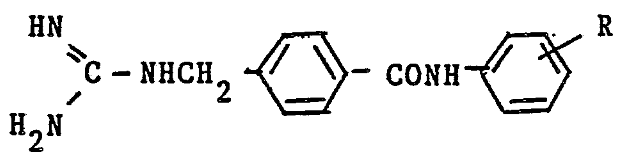

(I)

wherein R is a member selected from the group consisting of a halogen atom, a hydroxyl group, a lower alkyl group, a lower alkoxy group and a lower alkyl-substituted or unsubstituted carboxyl group,
or a salt thereof,
comprising the step of condensing together the compound of formula II, or optionally a salt thereof, and a compound of formula III, or optionally a salt thereof, in the presence of a dehydrating agent, and optionally converting the product into a salt.

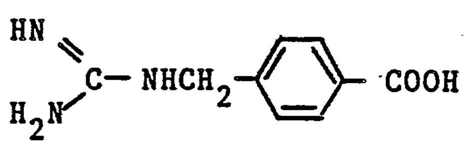

(II)

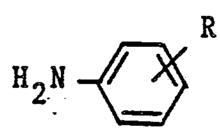

(III)

2. A process according to Claim 1 wherein the molar ratio of the compound of formula II to the compound of formula III is 1.0:1.0-1.5.

3. A process according to Claim 1 or Claim 2 wherein the dehydrating agent is dicyclohexylcarboiimide or phosphorus trichloride.

4. A process according to any preceding claim wherein the molar ratio of the compound of formula II to the dehydrating agent is 1.0:1.0-1.5.

5. A process according to any preceding claim wherein the condensation reaction is carried out for a period of from 10 to 80 hours at room temperature.

6. A process according to any of Claims 1-4 wherein the condensation reaction is carried out for a period of from 1 to 5 hours under reflux.

7. The use of a guanidinomethylbenzoic acid derivative of general formula:

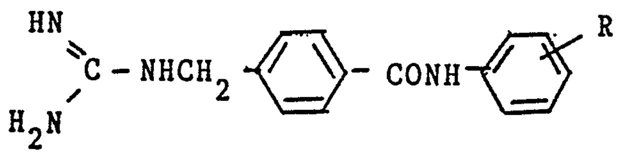

(I)

wherein R is a member selected from the group consisting of a halogen atom, a hydroxyl group, a lower alkyl group, a lower alkoxy group and a lower alkyl-substituted or unsubstituted carboxyl group,
or a salt thereof,
for the manufacture of a medicament for the treatment of ulcers.